# EUROPEAN PATENT APPLICATION

(11) **EP 3 682 790 A1**
(43) Date of publication of application: **22.07.2020**
(21) Application number: 18854577.6
(22) Date of filing: 07.09.2018
(51) Int. Cl.: A61B 1/045, A61B 1/00, A61B 1/055, A61B 1/12, G02B 23/26, H04N 7/18, H04N 13/111, H04N 13/117, H04N 13/337, H04N 13/383

(54) **ENDOSCOPE SYSTEM**

(30) Priority: 10.09.2017 JP 2017173598
(71) Applicant: Kairos Co., Ltd., Tokyo 105-0014 (JP)
(72) Inventor: CHIBA, Toshio, Tokyo 101-0062 (JP); YAMASHITA, Hiromasa, Tokyo 101-0062 (JP); TANIOKA, Kenkichi, Tokyo 101-0062 (JP); TAKAHASHI, Akira, Tokyo 101-0062 (JP)
(74) Representative: Becker, Eberhard
(86) International application number: PCT/JP2018/033245
(87) International publication number: WO 2019/049997

(57) **Abstract**

Provided is an endoscope system wherein, when a trigger signal generating unit of an endoscope 10 generates a zoom-in or zoom-out trigger signal, a control device 40 identifies the fixation point of an operator in a display image of a display device 30 on the basis of a relationship between a detection signal from a sensor in polarized glasses 50 and a detection signal from a sensor in the display device 30 at the time of generation of the trigger signal, and zooms in or zooms out the peripheries of the fixation point. As a result, convenience of the endoscope system including an 8K endoscope is further enhanced.

## Description

### BACKGROUND

### Technical Field

The invention relates to an endoscope system using an 8K high-resolution endoscope.

### Description of Related Art

Various techniques, related to a flexible endoscope for inserting an elongated insertion part into a body cavity and photographing the inside of the body cavity to perform minimally invasive surgery, have been proposed. Patent Document 1 is a document disclosing an invention related to this type of endoscope.

### Related Art

### Patent Document

[Patent Document 1] Japanese Laid-Open No. 2008-43763

### SUMMARY

### Problems to be Solved

With the development of image processing technology and optical technology, high-resolution imaging technologies called 4K and 8K have been put to practical use. The evolution of imaging technologies from 2K to 4K to 8K is also causing technological innovation in the field of medical equipment using endoscope and the field of minimally invasive surgery. When the 8K high-resolution imaging technology is applied to endoscope, for example, it becomes easy to recognize a fine thread for surgery, a fine affected part of an organ, and a boundary between organs and tissues, and it is also possible to observe at the cell level. As a result, the reliability and certainty of surgery are improved, and it is expected to see further progress in medical technology. That is, the discriminability of the affected part of an organ is enhanced, and the possibility of unexpectedly damaging parts other than the affected part is reduced. In addition, the field of view for surgery can be expanded, and surgery can be easily performed even when the surgery range is wide. It is also convenient for confirming the position of surgical equipment and avoiding interference between surgical equipment. Furthermore, it is possible for observation through a large screen so that all members involved in the surgery can share the same image to achieve smooth communication. Thus, the use of 4K and 8K high-resolution imaging technologies has great potential.

However, the conventional high-resolution endoscope system has room for improvement in terms of specifying the zoom position in the display image of the endoscope.

In view of such problems, the invention further improves the convenience of an endoscope system including an endoscope.

### Means for Solving the Problems

In order to solve the above problems, the invention provides an endoscope system that includes: an endoscope photographing a subject in a body cavity of a patient and outputting an image signal of a predetermined number of pixels; a control device performing a predetermined 3D process on an output signal of the endoscope and outputting a 3D image signal obtained by the 3D process to a display device as a moving image signal of a predetermined frame rate; polarized glasses worn by an operator performing a surgical operation on the patient; sensors provided in the display device and the polarized glasses respectively; and a trigger signal generating unit generating a trigger signal for instructing zoom of a display image of the display device. When the trigger signal generating unit generates the trigger signal, the control device identifies a fixation point of the operator in the display image of the display device based on a relationship between a detection signal of the sensor in the polarized glasses and a detection signal of the sensor in the display device at a time of generation of the trigger signal, and zooms in a periphery of the fixation point.

In the endoscope system, the sensors provided in the polarized glasses include a first sensor detecting a potential of a left nose pad of the polarized glasses, a second sensor detecting a potential of a right nose pad of the polarized glasses, a third sensor detecting a potential of a bridge of the polarized glasses, a fourth sensor detecting a position of the polarized glasses, and a fifth sensor detecting an orientation of lenses of the polarized glasses. The control device obtains a line-of-sight position corresponding to a line of sight of the operator on the lenses of the polarized glasses based on a potential waveform indicated by a detection signal of the first sensor, a potential waveform indicated by a detection signal of the second sensor, and a potential waveform indicated by a detection signal of the third sensor, and identifies the fixation point of the operator in the display display image based on the line-of-sight position, a relationship between a position where the display device is placed and a position detected by the fourth sensor, and a relationship between an orientation of the display device and an orientation detected by the fifth sensor.

In the endoscope system, the endoscope is an 8K endoscope, including: a housing; a solid-state imaging element housed in the housing and including pixels, a number of which corresponds to 8K and which each include a photoelectric conversion element, arranged in a matrix; and an insertion part extending with the housing as a base end, and the insertion part being inserted into the body cavity of the patient and guiding light from the subject in the body cavity to the solid-state imaging element. A pitch between adjacent pixels in the solid-state imaging element may be larger than a longest wavelength of wavelengths of light in illumination that illuminates the subject.

Further, the housing includes a mount part having a large cross-sectional area orthogonal to an optical axis of light passing through the insertion part, and a grip part having a smaller cross-sectional area than the mount part, and the solid-state imaging element may be housed in the mount part.

In addition, the insertion part includes a hollow rigid lens barrel, and a plurality of lenses including an objective lens may be provided in the rigid lens barrel.

Further, the endoscope system includes: an air supply pipe and an air exhaust pipe connected to the housing; an air supply and exhaust device forcibly supplying air into the housing via the air supply pipe and forcibly exhausting air from the housing via the air exhaust pipe; and an air cooling device cooling air flowing through the air supply pipe. The housing, the air supply pipe, and the air exhaust pipe are connected to form one closed space. In the housing, a first heat sink provided on the solid-state imaging element; a FPGA for image processing, a second heat sink provided on the FPGA, and a cover member covering the second heat sink and connected to the air exhaust pipe are provided. In the housing, a first airflow for cooling the first heat sink and a second airflow for cooling the second heat sink are generated. The first airflow may be formed by blowing cooling air supplied from the air supply pipe to the first heat sink to diverge around the first heat sink, and the second airflow may be formed to flow from around the second heat sink to the air exhaust pipe via the cover member.

In addition, the endoscope includes: a housing; a solid-state imaging element housed in the housing and including pixels, which each include a photoelectric conversion element, arranged in a matrix; and a hollow flexible lens barrel. In the flexible lens barrel, an objective lens, a multi-core fiber, and one or more mirrors for reflecting light from the subject one or more times and guiding the light to the objective lens are provided. At least one mirror of the one or more mirrors is tiltable around two axes, a first axis having a tilt with respect to an optical axis direction of light passing through each core of the multi-core fiber, and a second axis orthogonal to the first axis. The control device generates divided area images of different portions of the subject by periodically switching a tilt angle of the mirror at a time interval shorter than a frame switching time interval of the frame rate, and generates a moving image for one frame by combining the generated divided area images.

### BRIEF DESCRIPTION OF THE DRAWINGS

FIG. 1 is a diagram showing an overall configuration of an endoscope system according to an embodiment of the invention.
FIG. 2 is a diagram showing a state of an operation performed with the endoscope system.
FIG. 3 is a diagram showing characteristics of the endoscope system.
FIG. 4 is a diagram of a housing 131 of FIG. 1 when viewed from the direction of the arrow A.
FIG. 5 is a cross-sectional diagram of an insertion part 110 of FIG. 1 taken along the line B-B'.
FIG. 6 is a diagram showing polarized glasses 50 of FIG. 1.
FIG. 7 is a diagram showing a configuration of a control device 40 of FIG. 1.
FIG. 8 is a diagram showing processing of the control device 40 of FIG. 1.
FIG. 9 is a diagram showing processing of the control device 40 of FIG. 1.
FIG. 10 is a diagram showing an example of a waveform of a sensor of the polarized glasses 50 of FIG. 1.
FIG. 11 is a diagram showing an example of the waveform of the sensor of the polarized glasses 50 of FIG. 1.
FIG. 12 is a diagram showing an example of the waveform of the sensor of the polarized glasses 50 of FIG. 1.
FIG. 13 is a diagram showing an example of the waveform of the sensor of the polarized glasses 50 of FIG. 1.
   (A) and (B) of FIG. 14 are diagrams showing a configuration inside the housing 131 of the endoscope of FIG. 1 and a configuration of an air intake and exhaust device 60, an air cooling device 70, an air supply pipe 164A, and an air exhaust pipe 164B.
FIG. 15 is a diagram enlarging the area of a solid-state imaging element 1311 and a substrate 1312 of (A) of FIG. 14.
FIG. 16 is a diagram enlarging the area of an image processing FPGA 1331, a substrate 1332, a cover member 1338, and a duct 166B of (A) of FIG. 14.
FIG. 17 is a diagram showing a configuration of an endoscope system including a flexible endoscope 10' according to the second embodiment of the invention.
   (A) of FIG. 18 is a diagram of an insertion part 110' of FIG. 17 when viewed from the direction of the arrow A, and (B) of FIG. 18 is a cross-sectional diagram of the insertion part 110' taken along the line B-B'.
FIG. 19 is a diagram showing processing of a control device 40 of FIG. 17. (A) and (B) of FIG. 20 are diagrams showing characteristics of a modified example of the invention.

### DESCRIPTION OF THE EMBODIMENTS

### <First Embodiment>

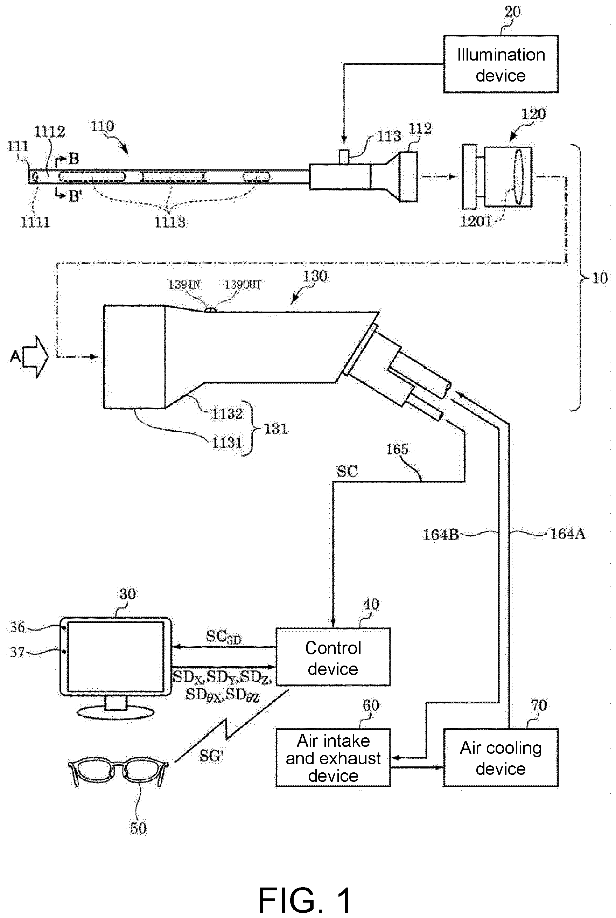

FIG. 1 is a diagram showing a configuration of an endoscope system including an endoscope according to the first embodiment of the invention. The endoscope system includes a rigid endoscope 10, an illumination device 20, a display device 30, a control device 40, polarized glasses 50, an air intake and exhaust device 60, and an air cooling device 70.

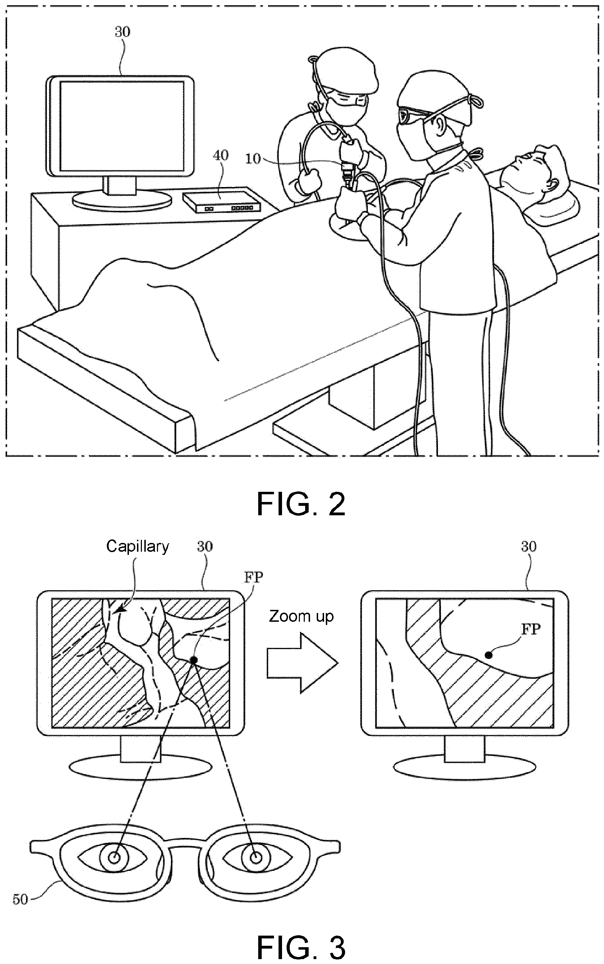

FIG. 2 is a diagram showing single-incision laparoscopic surgery, which is an example of a surgical operation performed with assistance of the endoscope system of the present embodiment. In single-incision laparoscopic surgery, an incision of about 2 cm is made in the abdomen of the patient (in most cases, the position of the navel) and a port (frame made of resin) is mounted to the incision, and carbon dioxide gas is introduced to expand the body cavity. The operator inserts an endoscope, a scalpel, and forceps (a surgical tool for pinching and pulling) into the body cavity from the port and treats the affected part in the body cavity while observing the image photographed by the endoscope, which is displayed on the display device 30.

In the present embodiment, the vein of the patient is injected with a near-infrared excitation drug prior to surgery. When this drug spreads over the body of the patient, the blood vessel illuminated by excitation light of a certain wavelength emits near-infrared light. In addition, the operator wears the polarized glasses 50 for stereoscopically viewing a 3D image. Then, as shown in FIG. 3, the control device 40 acquires from the rigid endoscope 10 a visible light image of the affected part in the body cavity of the patient and an infrared light image from the capillary in the depth, and displays these images as 3D moving images on the display device 30. Further, the control device 40 identifies a fixation point FP ahead of a line of sight of the operator in the display image of the display device 30 based on a relationship between a detection signal of a sensor in the polarized glasses 50 and a detection signal of a sensor in the display device 30, and zooms in the periphery of the fixation point FP.

In FIG. 1, the rigid endoscope 10 is a device that serves to photograph the body cavity of the patient. The rigid endoscope 10 has a camera body 130, an insertion part 110, and an eyepiece mount part 120. A housing 131 of the camera body 130 has a shape that the thickness of the cross section of the front part of the cylindrical body is increased.

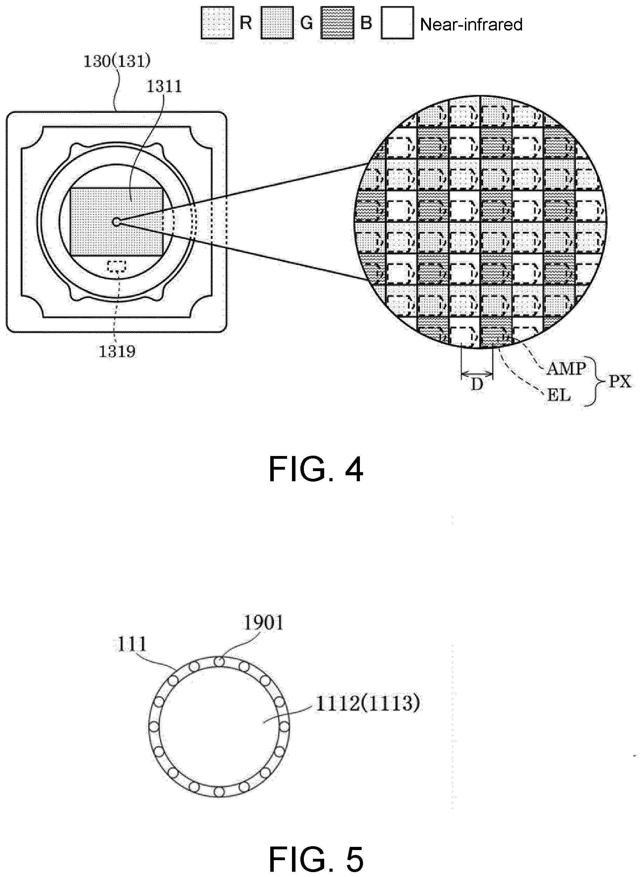

FIG. 4 is a diagram of the housing 131 of FIG. 1 when viewed from the direction of the arrow A. The housing 131 includes a mount part 1131 on the front side and a grip part 1132 on the rear side. A perfect circular opening is provided on the front surface of the mount part 1131. The area of the cross section of the mount part 1131 of the housing 131 is larger than the area of the grip part 1132 of the housing 131. An annular frame is fitted into the opening on the front surface of the mount part 1131 of the housing 131. At the position, facing the frame, on the front surface in the housing 131, a solid-state imaging element 1311 and an A/D conversion unit 1319 are provided. The solid-state imaging element 1311 is a CMOS (Complementary Metal Oxide Semiconductor) image sensor. The solid-state imaging element 1311 has pixels PXij (i = 1 to 4320, j = 1 to 7680), the number of which corresponds to 8K, arranged in a matrix. Here, i is the index of the pixel row, and j is the index of the pixel column. Each of the pixels PXij (i = 1 to 4320, j = 1 to 7680) of the solid-state imaging element 1311 has a photoelectric conversion element EL and an amplifier AMP that amplifies signal charge obtained by photoelectric conversion of the photoelectric conversion element EL.

In FIG. 4, the pixels PXij (i = 1 to 4320, j = 1 to 7680) in 4320 rows and 7680 columns in the solid-state imaging element 1311 form blocks each having four pixels PXij in 2 rows and 2 columns. Red, green, blue, and near-infrared filters are affixed to the four pixels PXij of each block. Specifically, the filter of the pixel PXij at the upper left of the block is a red filter (a filter that transmits only the red wavelength). The filter of the pixel PXij at the lower left of the block is a green filter (a filter that transmits only the green wavelength). The filter of the pixel PXij at the upper right of the block is a blue filter (a filter that transmits only the blue wavelength). The filter of the pixel PXij at the lower right of the block is a near-infrared filter (a filter that transmits only the near-infrared wavelength).

In addition, a pitch between adjacent pixels PXij in the solid-state imaging element 1311 (more specifically, a distance D between the centers of the light receiving areas of the photoelectric conversion elements EL of adjacent pixels PXij) is larger than the longest wavelength of the wavelengths of the light that illuminates the subject. If the light illuminating the subject contains only visible light, the pitch of the pixels PXij is preferably 2.8 µm to 3.8 µm. If the light illuminating the subject contains visible light and near-infrared light, the pitch of the pixels PXij is preferably 3.8 µm or more.

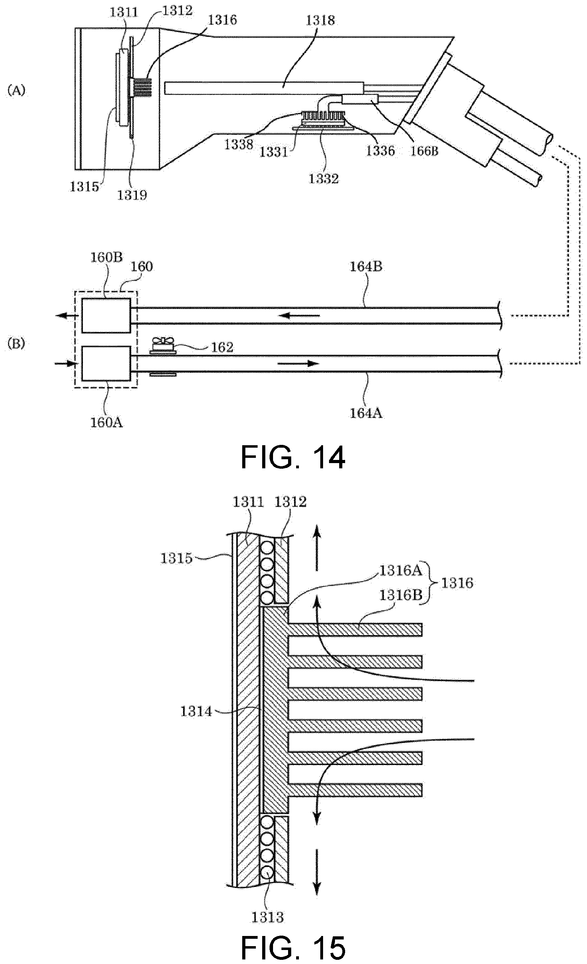

The A/D conversion unit 1319 dot-sequentially A/D converts the signal charges of the pixels PXij (i = 1 to 4320, j = 1 to 7680) that have been amplified by the amplifier AMP, and outputs the data obtained by the A/D conversion as an image signal SD. In the housing 131, in addition to the solid-state imaging element 1311 and the A/D conversion unit 1319, a heat sink 1316, a duct 1318, an image processing FPGA 1331, a heat sink 1336, a cover member 1338, a duct 166B, etc. are housed (see (A) and (B) of FIG. 14). Details of these parts will be described later.

Buttons 139IN and 139OUT are provided on the rear portion of a side surface of the housing 131. The buttons 139IN and 139OUT serve as a trigger generating unit. When the button 139IN is pressed shortly once, a zoom-in trigger signal that instructs zoom-in of the image displayed on the display device 30 is transmitted from the rigid endoscope 10 to the control device 40. When the button 139OUT is pressed shortly once, a zoom-out trigger signal that instructs zoom-out of the image displayed on the display device 30 is transmitted from the rigid endoscope 10 to the control device 40.

In FIG. 1, the eyepiece mount part 120 has a shape that a part of the outer periphery of the cylindrical body is recessed inward. An eyepiece 1201 is fitted into the eyepiece mount part 120. The insertion part 110 is a part inserted into the body cavity of the patient. The insertion part 110 has a rigid lens barrel 111 and an eyepiece 112. A connector 113 for the illumination device is provided at a position on the outer periphery of the insertion part 110 on the tip side of the eyepiece 112.

FIG. 5 is a cross-sectional diagram of the insertion part 110 of FIG. 1 taken along the line B-B'. As shown in FIG. 5, in the insertion part 110, a hollow light guide area 1112 is provided. The hollow light guide area 1112 is a cavity having a diameter slightly smaller than the diameter of the insertion part 110. Hundreds to thousands of optical fibers 1901 are embedded in the outer shell surrounding the hollow light guide area 1112 in the insertion part 110. FIG. 5 shows only 16 optical fibers 1901 for simplicity. A diffusion lens (not shown) is provided in front of the tips of the optical fibers in the insertion part 110. An objective lens 1111 is fitted at a position slightly inward of the tip in the hollow light guide area 1112 of the insertion part 110. A relay lens 1113 is fitted between the objective lens 1111 in the hollow light guide area 1112 and the eyepiece 112. The eyepiece 112 of the insertion part 110 is connected to the eyepiece mount part 120. The eyepiece mount part 120 is connected to the frame on the front surface of the housing 131.

In FIG. 1, the illumination device 20 includes a light-emitting element that emits light having a wavelength of visible light and a wavelength of near-infrared light, and a driver circuit that drives the light-emitting element. The illumination device 20 is connected to the connector 113 of the insertion part 110. Illumination light (light having wavelengths of visible light and near-infrared light) of the illumination device 20 passes through the optical fibers 1901 of the insertion part 110 and is emitted into the body cavity via the diffusion lens ahead of it.

In FIG. 1, the display device 30 is a liquid crystal display having display pixels corresponding to 8K (display pixels of 4320 rows and 7680 columns). The display device 30 is provided with a position detection sensor 36 and an orientation detection sensor 37. The position detection sensor 36 detects the position of the display device 30. Specifically, the position detection sensor 36 outputs coordinate signals SD_{X}, SD_{Y}, and SD_{Z} indicating the position of the display device 30 on the X axis, the position of the display device 30 on the Y axis, and the position of the display device 30 on the Z axis when setting the direction parallel to the earth axis direction as the Z-axis direction, one direction orthogonal to the Z-axis direction as the Y-axis direction, a direction orthogonal to both the Z-axis direction and the Y-axis direction as the X-axis direction, and a reference point in the room for surgery (for example, the center of the room) as the origin (0.0.0).

The orientation detection sensor 37 detects the orientation of the display device 30. Specifically, the orientation detection sensor 37 sets a plane parallel to the X-axis direction and the Y-axis direction as the reference plane, sets a tilt of the display screen of the display device 30 in a direction around the X axis with respect to the reference plane as an elevation angle of the display screen, and outputs an angle signal SDex indicating the elevation angle of the display screen. Further, the orientation detection sensor 37 sets a tilt of the display screen of the display device 30 in a direction around the Z axis with respect to the reference plane as the direction of the display screen, and outputs an angle signal SDez indicating the direction of the display screen.

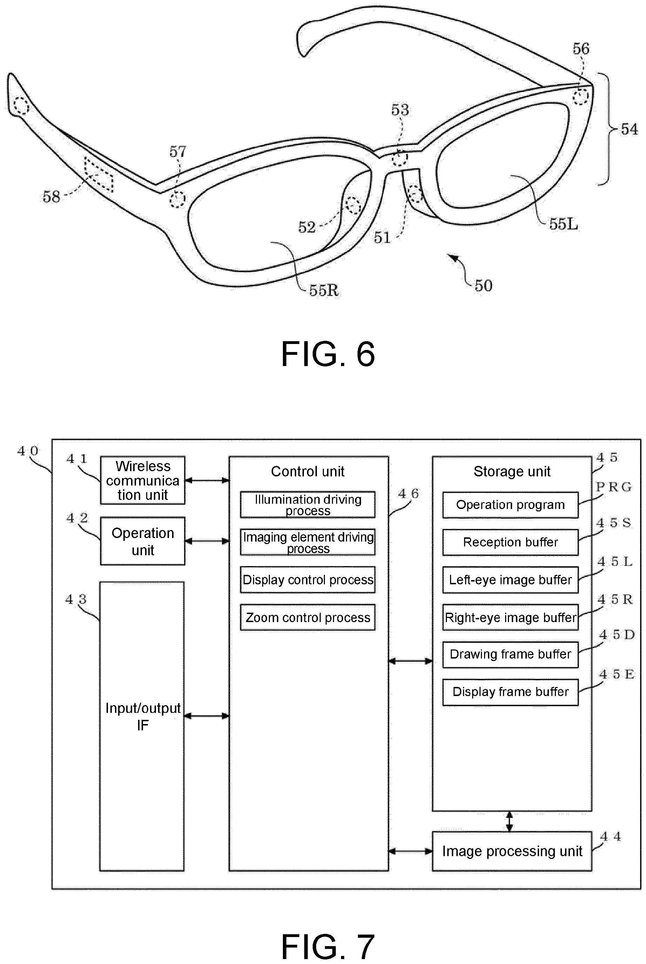

In FIG. 1, the polarized glasses 50 are passive (circularly polarized filter type) polarized glasses. As shown in FIG. 6, a left lens 55L and a right lens 55R are fitted into a frame 54 of the polarized glasses 50. The left lens 55L guides a left-eye image of the 3D image to the left-eye retina of the operator. The right lens 55R guides a right-eye image of the 3D image to the right-eye retina of the operator.

A position detection sensor 56 is embedded at the upper left of the left lens 55L in the frame 54 of the polarized glasses 50. The position detection sensor 56 detects the position of the polarized glasses 50. More specifically, the position detection sensor 56 outputs coordinate signals SGx, SG_{Y}, and SGz indicating the positions of the polarized glasses 50 on the X axis, the Y axis, and the Z axis when setting the reference point (the center of the room) as the origin (0.0.0).

An orientation detection sensor 57 is embedded at the upper right of the right lens 55R in the frame 54 of the polarized glasses 50. The orientation detection sensor 57 detects the orientations of the lenses 55L and 55R of the polarized glasses 50. Specifically, the orientation detection sensor 57 sets a tilt of the lenses 55L and 55R of the polarized glasses 50 in the direction around the X axis with respect to the reference plane (a plane parallel to the X-axis direction and the Y-axis direction) as an elevation angle of the lenses 55L and 55, and outputs an angle signal SGex indicating the elevation angle of the lenses 55L and 55R. In addition, the orientation detection sensor 57 sets a tilt in the direction around the Z axis with respect to the reference plane as the direction of the lenses 55L and 55R, and outputs an angle signal SGez indicating the direction of the lenses 55L and 55R.

A first potential sensor 51 is embedded in the left nose pad of the frame 54 of the polarized glasses 50. A second potential sensor 52 is embedded in the right nose pad of the frame 54 of the polarized glasses 50. A third potential sensor 53 is embedded in the middle bridge of the frame 54 of the polarized glasses 50. The potential sensor 51 detects a potential of a portion of the face of the operator with which the left nose pad is in contact, and outputs a left potential signal SG_{V1} indicating the detected potential. The potential sensor 52 detects a potential of a portion of the face of the operator with which the right nose pad is in contact, and outputs a right potential signal SGv2 indicating the detected potential. The potential sensor 53 detects a potential of a portion of the face of the operator with which the bridge is in contact, and outputs an upper potential signal SGv3 indicating the detected potential.

A wireless communication unit 58 is embedded in the right temple of the frame 54 of the polarized glasses 50. The wireless communication unit 58 modulates a carrier by the output signals SG_{X}, SG_{Y}, and SG_{Z} of the position detection sensor 56, the output signals SGex and SGez of the orientation detection sensor 57, the output signal SG_{V1} of the potential sensor 51, the output signal SGv2 of the potential sensor 52, and the output signal SGv3 of the potential sensor 53, and transmits a radio signal SG' obtained by the modulation.

In FIG. 1, the control device 40 is a device that serves as the control center of the endoscope system. As shown in FIG. 7, the control device 40 includes a wireless communication unit 41, an operation unit 42, an input/output interface 43, an image processing unit 44, a storage unit 45, and a control unit 46. The wireless communication unit 41 receives the radio signal SG' and supplies signals SG_{X}, SG_{Y}, SG_{Z}, SG_{θX}, SG_{θZ}, SG_{V1}, SG_{V2}, and SG_{V3} obtained by demodulating the signal SG' to the control unit 46.

The operation unit 42 is a device that performs various operations such as a keyboard, a mouse, a button, and a touch panel. The input/output interface 43 mediates transmission and reception of data between the display device 30 and the rigid endoscope 10 and the control device 40. The image processing unit 44 is an image processor. The storage unit 45 has both a volatile memory such as a RAM (Random Access Memory) and a non-volatile memory such as an EEPROM (Electrically Erasable Programmable Read Only Memory). The storage unit 45 stores an operation program PRG of the control unit 46 or the image processing unit 44. In addition, the storage unit 45 provides the control unit 46 and the image processing unit 44 with storage areas and work areas such as a reception buffer 45S, a left-eye image buffer 45L, a right-eye image buffer 45R, a drawing frame buffer 45D, and a display frame buffer 45E.

The control unit 46 includes a CPU. The control unit 46 executes an illumination driving process, an imaging element driving process, a display control process, and a zoom control process by running of the operation program PRG in the storage unit 45. The illumination driving process is a process of supplying a drive signal for driving a driver in the illumination device 20 to the illumination device 20 via the input/output interface 43. The imaging element driving process is a process of supplying a drive signal for driving the solid-state imaging element 1311 in the rigid endoscope 10 to the endoscope 10 via the input/output interface 43.

The display control process is a process of applying a 3D process on the image signal SD transmitted from the rigid endoscope 10 and outputting the 3D image obtained by the 3D process to the display device 30 as an image signal SD_{3D} of a moving image having a frame rate of 59.94 frames per second.

The zoom control process is a process of obtaining a line-of-sight position corresponding to the line of sight of the operator on the lenses 55L and 55R of the polarized glasses 50 based on the potential waveform indicated by the detection signal SG_{V1} of the potential sensor 51, the potential waveform indicated by the detection signal SGv2 of the potential sensor 52, and the potential waveform indicated by the detection signal SGv3 of the potential sensor 53 of the polarized glasses 50 at the time of generation of a zoom-in or zoom-out trigger signal when the trigger signal is generated, identifying the fixation point FP of the operator based on the line-of-sight position, the relationship between the position of the display device 30 and the position of the polarized glasses 50, and the relationship between the orientation of the display device 30 and the orientation of the polarized glasses 50, and enlarging or reducing the image of the periphery of the fixation point FP.

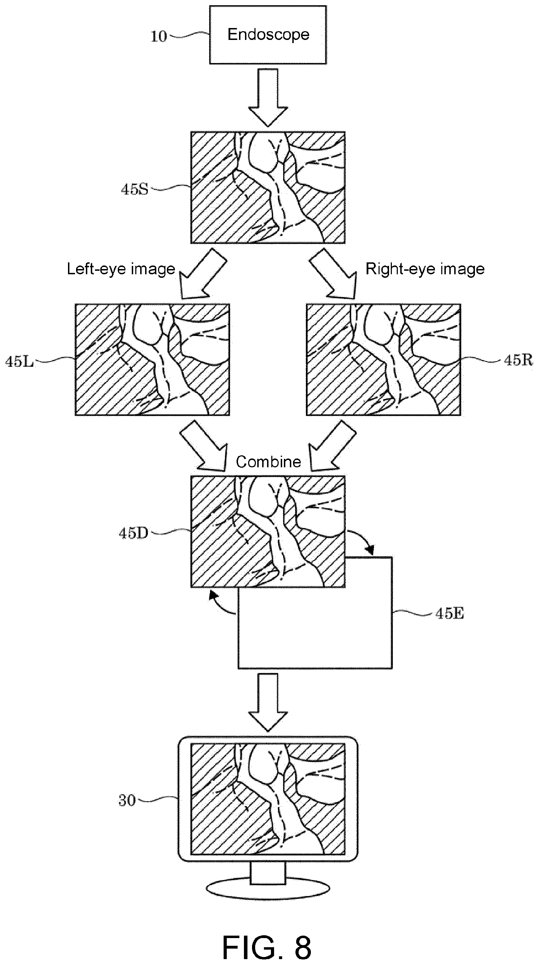

More specifically, as shown in FIG. 8, in the display control process, the control unit 46 stores the image signal SD (the image of visible light and the image of near-infrared light) in the reception buffer 45S of the storage unit 45 as photographed image data. Next, the control unit 46 generates left-eye image data and right-eye image data having binocular parallax from the photographed image data in the reception buffer 45S, and stores the left-eye image data and the right-eye image data in the left-eye image buffer 45L and the right-eye image buffer 45R. Then, the control unit 46 combines the left-eye image data and the right-eye image data, and stores the combined image data in the drawing frame buffer 45D of the storage unit 45. The control unit 46 replaces the drawing frame buffer 45D and the display frame buffer 45E of the storage unit 45 every 1/59.94 seconds (≒ 0.17 seconds), and outputs the image data in the display frame buffer 45E to the display device 30 as the image signal SD_{3D}.

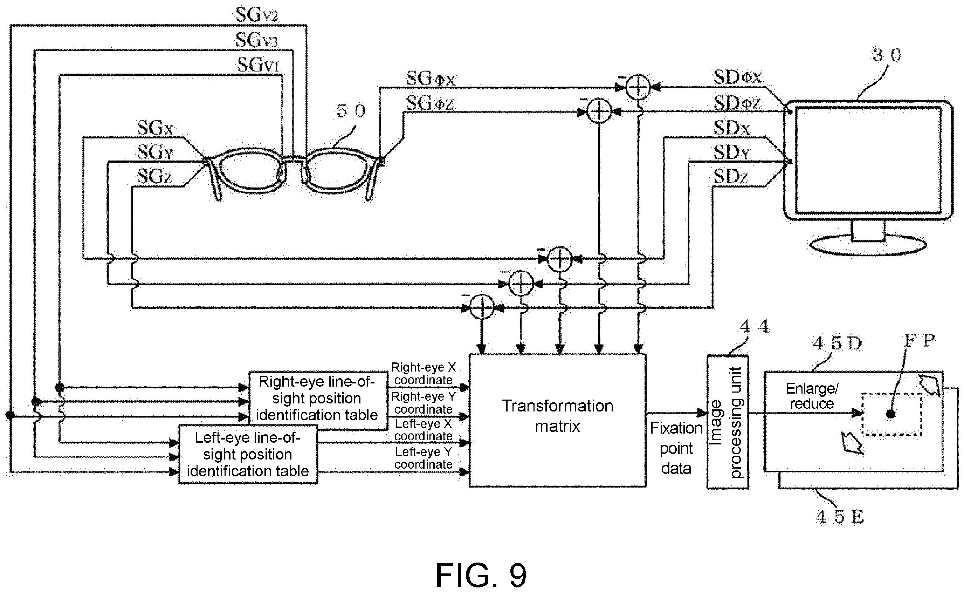

As shown in FIG. 9, in the zoom control process, the control unit 46 obtains, from the output signal SG_{V1} of the potential sensor 51, the output signal SGv2 of the potential sensor 52, and the output signal SGv3 of the potential sensor 53 of the polarized glasses 50, the potential of the potential sensor 51 (an absolute value of the amplitude and a positive/negative sign) when the potential of the potential sensor 53 is set as the reference potential and the potential of the potential sensor 52 (an absolute value of the amplitude and a positive/negative sign) when the potential of the potential sensor 53 is set as the reference potential.

Then, the control unit 46 identifies the X coordinate value and Y coordinate value of the left-eye line-of-sight position of the operator on the lens 55L of the polarized glasses 50 (X coordinate value and Y coordinate value of the intersection position of the XY plane, which is parallel to the lens 55L and takes the position of the potential sensor 53 as the origin (0.0), and the line of sight of the left eye) with reference to a left-eye line-of-sight position identification table of the storage unit 45. Further, the control unit 46 identifies the X coordinate value and Y coordinate value of the right-eye line-of-sight position of the operator on the lens 55R of the polarized glasses 50 (X coordinate value and Y coordinate value of the intersection position of the XY plane, which is parallel to the lens 55R and takes the position of the potential sensor 53 as the origin (0.0), and the line of sight of the right eye) with reference to a right-eye line-of-sight position identification table of the storage unit 45.

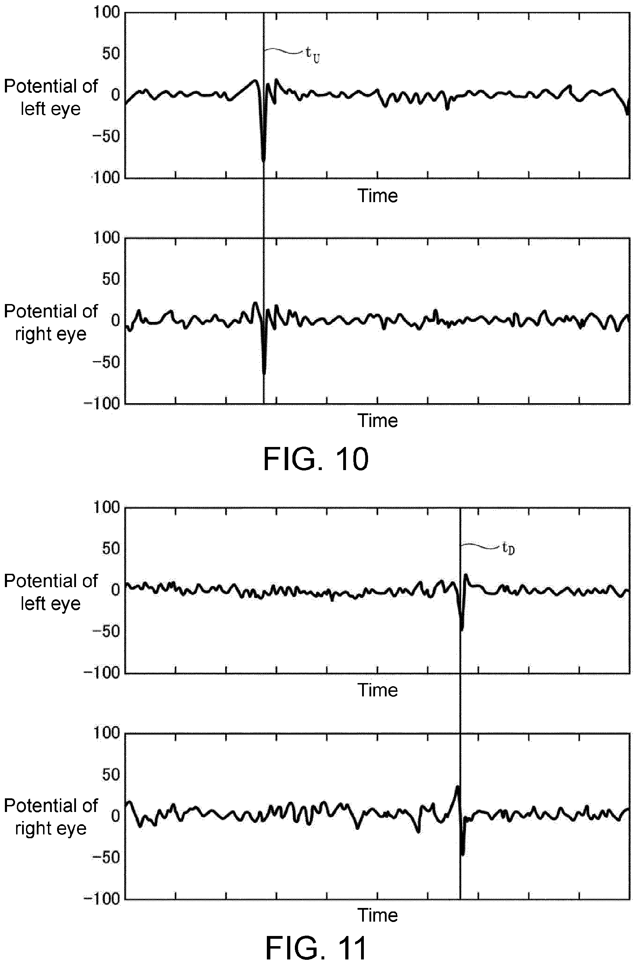

Here, for human eyes, the cornea side is positively charged and the retina side is negatively charged. Therefore, as indicated by the waveform of FIG. 10, when the operator turns the line of sight upward from the front, the potential (left-eye potential) of the potential sensor 51 taking the potential of the potential sensor 53 as reference and the potential (right-eye potential) of the potential sensor 52 taking the potential of the potential sensor 53 as reference at the time when the line of sight is turned upward (time t_{U} of FIG. 10) both become negative.

As shown in FIG. 11, when the operator turns the line of sight downward from the front, the potential (left-eye potential) of the potential sensor 51 taking the potential of the potential sensor 53 as reference and the potential (right-eye potential) of the potential sensor 52 taking the potential of the potential sensor 53 as reference at the time when the line of sight is turned downward (time t_{D} of FIG. 11) both become positive.

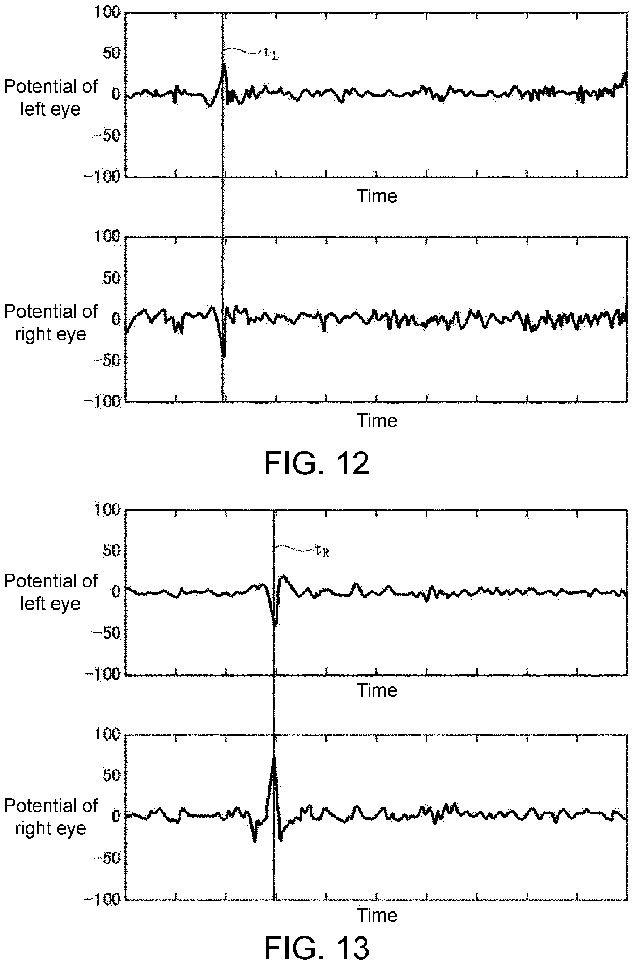

As shown in FIG. 12, when the operator turns the line of sight from the front to the left, the potential (left-eye potential) of the potential sensor 51 taking the potential of the potential sensor 53 as reference at the time when the line of sight is turned to the left (time t_{L} of FIG. 12) becomes positive, and the potential (right-eye potential) of the potential sensor 52 taking the potential of the potential sensor 53 as reference becomes negative.

As shown in FIG. 13, when the operator turns the line of sight from the front to the right, the potential (left-eye potential) of the potential sensor 51 taking the potential of the potential sensor 53 as reference at the time when the line of sight is turned to the left (time t_{R} of FIG. 13) becomes negative, and the potential (right-eye potential) of the potential sensor 52 taking the potential of the potential sensor 53 as reference becomes positive.

In the left-eye line-of-sight position identification table, the measured values of the potentials of the first potential sensor 51, the second potential sensor 52, and the third potential sensor 53 when the line of sight of the subject is directed to each point on the left lens 55L of the polarized glasses 50 are recorded in association with the X coordinate and Y coordinate of each point. In the right-eye line-of-sight position identification table, the measured values of the potentials of the first potential sensor 51, the second potential sensor 52, and the third potential sensor 53 when the line of sight of the subject wearing the polarized glasses 50 is directed to each point on the right lens 55R are recorded in association with the X coordinate and Y coordinate of each point. Therefore, by referring to the tables based on the output signals SG_{V1}, SGv2, and SGv3 of the sensors of the polarized glasses 50 of the operator, the line-of-sight position of the operator on the lenses of the polarized glasses 50 can be identified.

In FIG. 9, the control unit 46 respectively obtains the difference SDx-SGx between the output signal SDx of the position detection sensor 36 of the display device 30 and the output signal SGx of the position detection sensor 56 of the polarized glasses 50, the difference SD_{Y}-SG_{Y} between the output signal SD_{Y} of the position detection sensor 36 of the display device 30 and the output signal SG_{Y} of the position detection sensor 56 of the polarized glasses 50, the difference SDz-SGz between the output signal SDz of the position detection sensor 36 of the display device 30 and the output signal SGz of the position detection sensor 56 of the polarized glasses 50, the difference SDex-SGex between the output signal SDex of the orientation detection sensor 37 of the display device 30 and the output signal SGex of the orientation detection sensor 57 of the polarized glasses 50, and the difference SDez-SGez between the output signal SD_{θZ} of the orientation detection sensor 37 of the display device 30 and the output signal SGez of the orientation detection sensor 57 of the polarized glasses 50.

From the differences SD_{X}-SG_{X}, SD_{Y}-SG_{Y}, SD_{Z}-SG_{Z}, SD_{θX}-SG_{θX}, and SD_{θZ}-SG_{θZ}, the control unit 46 generates a transformation matrix for transforming the X coordinate values and Y coordinate values of the line-of-sight positions of the left and right eyes into the X coordinate value and Y coordinate value of the fixation point FP on the display screen of the display device 30, and obtains the X coordinate value and Y coordinate value of the fixation point FP by applying the transformation matrix to the X coordinate values and Y coordinate values of the line-of-sight positions of the left and right eyes. The control unit 46 supplies the X coordinate value and Y coordinate value of the fixation point FP to the image processing unit 44 as fixation point data. If a zoom-in trigger signal is generated, when receiving the fixation point data, the image processing unit 44 performs a process of rewriting the image in the drawing frame buffer 45D to an enlarged image of a predetermined rectangular area centered on the fixation point FP. In addition, if a zoom-out trigger signal is generated, when receiving the fixation point data, the image processing unit 44 performs a process of rewriting the image in the drawing frame buffer 45D to a reduced image of the predetermined rectangular area centered on the fixation point FP.

In FIG. 1, a cable 165, an air supply pipe 164A, and one end of an air exhaust pipe 164B are connected to the rear end of the grip part 1132 of the housing 131 of the endoscope. The cable 165 is connected to the control device 40. The air supply pipe 164A is connected to the air intake and exhaust device 60 via the air cooling device 70. The air exhaust pipe 164B is connected to the air intake and exhaust device 60. The air intake and exhaust device 60 is a device that serves to forcibly supply air into the housing 131 via the air supply pipe 164A as well as forcibly exhaust air from inside the housing 131 via the air exhaust pipe. The air cooling device 70 is a device that serves to cool the air flowing through the air supply pipe 164A.

The housing 131 of the rigid endoscope 10, the air supply pipe 164A, and the air exhaust pipe 164B form one closed space, and a flow of air for cooling the inside of the housing 131 is generated in the closed space. The flow of air will be described. (A) of FIG. 14 is a diagram showing the details of the configuration inside the housing 131. (B) of FIG. 14 is a diagram showing the details of the configuration of the air intake and exhaust device 60, the air cooling device 70, the air supply pipe 164A, and the air exhaust pipe 164B.

As shown in (A) of FIG. 14, the solid-state imaging element 1311 and a substrate 1312 supporting the solid-state imaging element 1311 are provided at a position facing the eyepiece 1201 in the front of the housing 131. Electronic components such as the A/D conversion unit 1319 are mounted on the substrate 1312. FIG. 15 is a diagram enlarging the area of the solid-state imaging element 1311 and the substrate 1312 of (A) of FIG. 14. An anti-reflection glass 1315 is attached to the solid-state imaging element 1311. A plurality of ball grids 1313 are interposed between the solid-state imaging element 1311 and the substrate 1312.

A heat sink 1316 is provided behind the substrate 1312. The heat sink 1316 is of a so-called needle type in which a plurality of fins 1316B are erected from a flat plate 1316A. An opening having substantially the same size as the flat plate 1316A of the heat sink 1316 is provided at the center of the substrate 1312. The flat plate 1316A of the heat sink 1316 is fitted into the opening. The flat plate 1316A of the heat sink 1316 is bonded to the solid-state imaging element 1311 via a heat conductive adhesive 1314. A duct 1318 is provided at a position on the inner side of the grip part 1132 in the housing 131. One end of the duct 1318 is directed to the fins 1316B of the heat sink 1316. The other end of the duct 1318 is connected to the air supply pipe 164A.

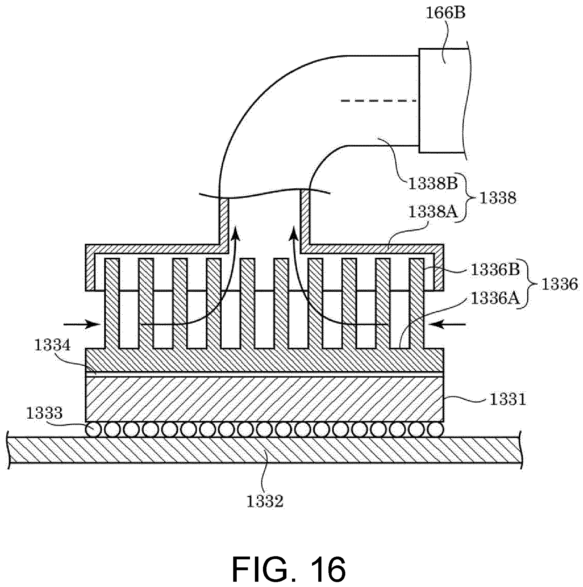

A structure composed of the image processing FPGA (Field Programmable Gate Array) 1331, a substrate 1332, a heat sink 1336, a cover member 1338, and a duct 166B is provided below the duct 1318 on the inner side of the grip part 1132 of the housing 131. FIG. 16 is a diagram enlarging the area of the image processing FPGA 1331, the substrate 1332, the cover member 1338, the heat sink 1336, and the duct 166B of (A) of FIG. 14. A plurality of ball grids 1333 are interposed between the image processing FPGA 1331 and the substrate 1332.

The heat sink 1336 is provided above the substrate 1332. The heat sink 1336 is of a so-called needle type in which a plurality of fins 1336B are erected from a flat plate 1336A. The size of the flat plate 1336A of the heat sink 1336 is substantially the same as the size of the image processing FPGA 1331. The flat plate 1336A of the heat sink 1336 is bonded to the image processing FPGA 1331 via a heat conductive adhesive 1334.

The cover member 1338 is provided above the heat sink 1336. The cover member 1338 has a shape that opens the lower surface of a thin box 1338A, protrudes a cylinder 1338B from the center of a surface opposite to the opened side, and mildly bends the cylinder 1338B in a direction orthogonal to the base end surface of the cylinder. The opening of the cover member 1338 covers the heat sink 1336. The tip of the cylinder 1338B of the cover member 1338 is connected to the duct 166B. The other end of the duct 166B is connected to the air exhaust pipe 164B.

In the above configuration in the housing 131, by operating the air supply device 160A and the air exhaust device 160B in the air intake and exhaust device 60 and the air cooling device 70, the air supply device 160A generates a positive pressure of +10 hPa to +20 hPa, and sends out the air sucked in from the outside to the air supply pipe 164A by this pressure. The air exhaust device 160B generates a negative pressure of -10 hPa to -20 hPa, and sends out the air sucked in from the air exhaust pipe 164B164B to the outside by this pressure.

The air sent from the air supply device 160A to the air supply pipe 164A is cooled by the air cooling device 70 when passing through the air cooling device 70. The cooled air passes through the air supply pipe 164A and the duct 1318, and is blown to the heat sink 1316 as a first airflow from the opening at the tip of the duct 1318. The first airflow passes through the heat sink 1316 and flows to the side part thereof, and circulates in housing 131. The first airflow takes away the heat as it passes through the heat sink 1316. The air that passes through the heat sink 1316 and flows to the lower part thereof is blown to the lower heat sink 1336 in the housing 131 as a second airflow. After passing through the heat sink 1336, the second airflow is sucked into the opening of the cover member 1338. The second airflow takes away the heat as it passes through the heat sink 1336. The air that passes through the heat sink 1336 and is sucked into the cover member 1338 is exhausted to the outside by the air exhaust device 160B via the duct 166B and the air exhaust pipe 164B.

The above are the details of the present embodiment. According to the present embodiment, the following effects can be obtained. First, in the present embodiment, the solid-state imaging element 1311 of the rigid endoscope 10 has the pixels PXij (i = 1 to 4320, j = 1 to 7680), the number of which corresponds to 8K. Here, for the conventional 2K or 4K endoscope, the endoscope must be brought close to the subject for photographing, and the surgical instruments such as a scalpel and forceps may interfere with the endoscope in the body cavity and cause the surgery to be delayed. However, the endoscope of the present embodiment can obtain a sufficiently fine photographed image even if the subject is photographed from a position about 8 cm to 12 cm away from the subject in the body cavity. Therefore, a wide field of view and a wide surgical space in the body cavity can be ensured, and the surgery can be realized more smoothly.

Second, in the present embodiment, a red filter, a green filter, a blue filter, and a near-infrared filter are attached to the pixels PXij (i = 1 to 4320, j = 1 to 7680) of the solid-state imaging element 1311 of the rigid endoscope 10, and the display device 30 displays an image in which a RGB image of visible light and an image of near-infrared light are superimposed. Here, when an infrared excitation drug is injected into the vein of the patient, the drug binds to the protein in the blood. When excitation light is applied from outside the blood vessel, near-infrared light is emitted and the near-infrared light is reflected in the image. Thus, the operator can simultaneously grasp the state of the affected part itself, which is the target to be treated, and the state of distribution of blood vessel in the depth by viewing one image displayed on the display device 30.

Third, in the present embodiment, the display device 30 displays the photographed image of the rigid endoscope 10 as a 3D moving image. Thus, the operator can accurately grasp the positional relationship and the distance between the target organ that is to be treated and the surgical instruments in the body cavity.

Fourth, in the present embodiment, the control device 40 identifies the fixation point FP of the operator in the display image of the display device 30 based on the relationship between the detection signal of the sensor in the polarized glasses 50 and the detection signal of the sensor in the display device 30 at the time of generation of the trigger signal, and enlarges and displays the periphery of the fixation point FP. The operator can specify the zoom-in or zoom-out range as intended without performing a troublesome input operation.

Fifth, in the present embodiment, the pitch between adjacent pixels PXij in the solid-state imaging element 1311 of the rigid endoscope 10 is larger than the longest wavelength of the wavelengths of the light in the illumination that illuminates the subject. Here, the 8K solid-state imaging element 1311 is an array of 4320 rows and 7680 columns of the pixels PXij (i = 1 to 4320, j = 1 to 7680). Therefore, if the degree of integration of the pixels PXij (i = 1 to 4320, j = 1 to 7680) is increased, it is difficult to make the size of the housing 131 easy to handle. On the other hand, if the degree of integration of the pixels PXij (i = 1 to 4320, j = 1 to 7680) of the solid-state imaging element 1311 is too high, the relationship between the pitch between adjacent pixels PXij in the solid-state imaging element 1311 and the wavelength of light becomes "pitch < wavelength", and due to the diffraction effect of light, the photographed image may be blurred. By making the pitch between adjacent pixels PXij in the solid-state imaging element 1311 larger than the longest wavelength of the wavelengths of the light in the illumination that illuminates the subject, it is possible to provide an endoscope that achieves both a clear image and compactness.

Sixth, the housing 131 of the rigid endoscope 10 includes the mount part 1131 having a large cross-sectional area orthogonal to the optical axis of the light passing through the insertion part 110, and the grip part 1132 having a smaller cross-sectional area than the mount part 1131. Since the 8K solid-state imaging element 1311 has 4320 rows and 7680 columns of the pixels PXij (i = 1 to 4320, j = 1 to 7680), it is difficult to make the size the same as that of a 4K imaging element, and there are some limits to miniaturization. If the overall thickness of the housing of the 8K endoscope is adjusted to the vertical and horizontal dimensions of the solid-state imaging element 1311, the endoscope cannot be held with one hand. In the present embodiment, the cross-sectional area of the grip part 1132 is smaller than the cross-sectional area of the mount part 1131, so it is possible to provide an endoscope which has a high resolution and can be held with one hand to be handled properly.

Seventh, in the present embodiment, the housing 131 of the rigid endoscope 10 is provided with the first heat sink 1316 provided on the solid-state imaging element 1311, the image processing FPGA 1331, the second heat sink 1336 provided on the FPGA 1331, and the cover member 1338 that covers the second heat sink 1336 and is connected to the air exhaust pipe 164B. Further, in the present embodiment, the first airflow for cooling the first heat sink 1316 and the second airflow for cooling the second heat sink 1336 are generated. The first airflow is formed by blowing cooling air supplied from the air supply pipe 164A to the first heat sink 1316 to diverge around the first heat sink 1316, and the second airflow is formed to flow from around the second heat sink 1336 to the air exhaust pipe 164B via the cover member 1338. Therefore, the solid-state imaging element 1311 and the image processing FPGA 1331, which are the main heat sources in the housing 131 of the endoscope, can be efficiently cooled.

### <Second Embodiment>

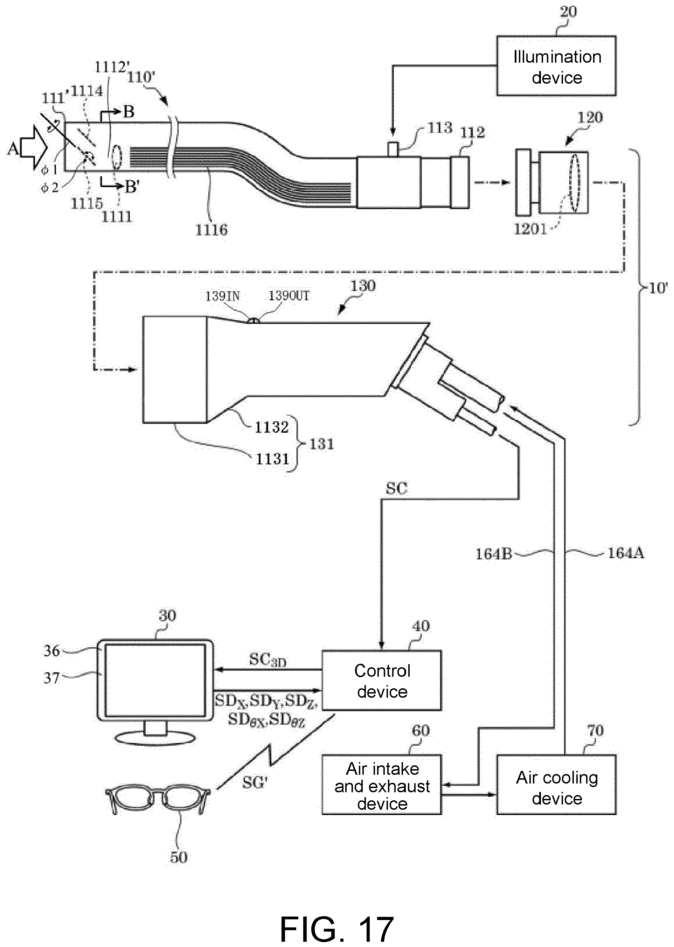

FIG. 17 is a diagram showing a configuration of an endoscope system including a flexible endoscope 10' according to the second embodiment of the invention. The endoscope system of the present embodiment supports surgery performed by inserting the flexible endoscope 10' from the mouth or anus for treatment on an organ in the body cavity. In the present embodiment, the rigid endoscope 10 of the endoscope system of the first embodiment is replaced with the flexible endoscope 10'. The solid-state imaging element in the housing 131 of the flexible endoscope 10' has a smaller number of pixels (for example, a solid-state imaging element having 300,000 pixels) than that of the solid-state imaging element in the housing 131 of the rigid endoscope 10 of the first embodiment.

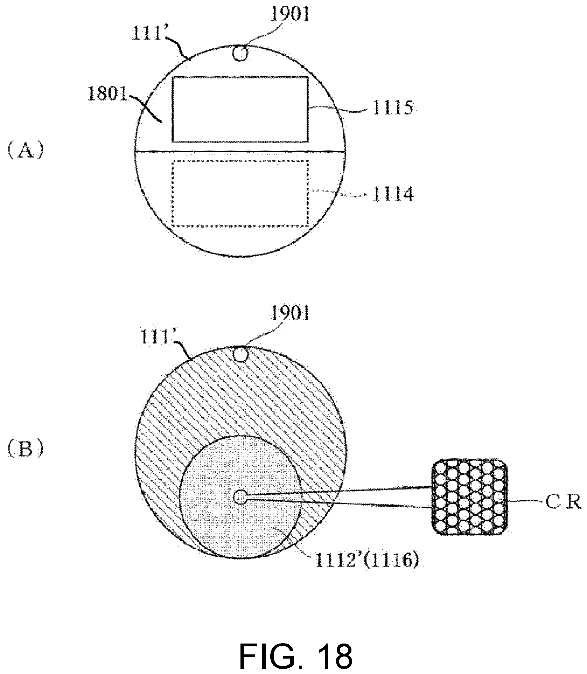

In FIG. 17, elements the same as those of FIG. 1 are denoted by the same reference numerals, and elements different from those of FIG. 1 are denoted by different reference numerals. (A) of FIG. 18 is a diagram of an insertion part 110' of FIG. 1 when viewed from the direction of the arrow A, and (B) of FIG. 18 is a cross-sectional diagram of the insertion part 110' of FIG. 1 taken along the line B-B'. The insertion part 110' of the flexible endoscope 10' has a flexible lens barrel 111' and an eyepiece 112. The flexible lens barrel 111' is made of a flexible material.

A hollow light guide area 1112' is provided in the flexible lens barrel 111' of the insertion part 110'. The hollow light guide area 1112' is a cavity having a diameter that is about half the diameter of the insertion part 110'. The center of the cross section of the hollow light guide area 1112' is shifted from the center of the cross section of the insertion part 110'. The portion of the outer shell surrounding the hollow light guide area 1112' of the insertion part 110' on the side close to the center of the hollow light guide area 1112 is thinner, and the portion on the side close to the center of the hollow light guide area 1112' is thicker. One optical fiber 1901 is embedded in the thick portion of the outer shell of the insertion part 110'. A diffusion lens (not shown) is embedded in front of the tip of the optical fiber 1901 in the insertion part 110'.

An objective lens 1111 is fitted at a position that is farther from the tip in the hollow light guide area 1112' of the insertion part 110. A multi-core fiber 1116 is housed between the objective lens 1111 and the eyepiece 112 in the hollow light guide area 1112'. The light guiding direction of each core CR of the multi-core fiber 1116 is parallel to the direction in which the insertion part 110' extends.

The cross section of the portion on the tip side of the position where the objective lens 1111 is fitted in the hollow light guide area 1112' of the insertion part 110 is wider than the cross section of the portion where the objective lens 1111 and the multi-core fiber 1116 are provided. A movable mirror 1114 is supported at a position facing the objective lens 1111 in the portion on the tip side of the hollow light guide area 1112'. The movable mirror 1114 is a MEMS (Micro Electro Mechanical Systems) mirror. The movable mirror 1114 is supported to be swingable around two axes, a first axis ϕ1 and a second axis ϕ2. The first axis ϕ1 is an axis that intersects the optical axis of the light passing through each core of the multi-core fiber 1116 with a tilt with respect to the optical axis (the optical axis of the objective lens 1111). The second axis ϕ2 is an axis orthogonal to both the optical axis of the objective lens 1111 and the first axis ϕ1. A fixed mirror 1115 is fixed between the movable mirror 1114 and the optical fiber 1901 in the portion on the tip side of the hollow light guide area 1112'. A reflective surface of the movable mirror 1114 faces the objective lens 1111 and the fixed mirror 1115. A reflective surface of the fixed mirror 1115 faces the movable mirror 1114 and the outside of the opening 1801 at the tip of the insertion part 110'.

In the present embodiment, the control unit 46 of the control device 40 performs an illumination driving process, an imaging element driving process, a display control process, a zoom control process, and a mirror driving process by running of the operation program PRG in the storage unit 45. The mirror driving process is a process of supplying a drive signal for driving the movable mirror 1114 to the movable mirror 1114 via the input/output interface 43. The control unit 46 generates divided area images of different portions of the subject in the body cavity by periodically switching the tilt angles of the movable mirror 1114 around the axis ϕ1 and the axis ϕ2 at intervals of a time T (for example, T = 1/120 second) shorter than the frame switching of the frames of the moving image through supply of a drive signal to the movable mirror 1114, and generates the image of a frame by combining the generated divided area images.

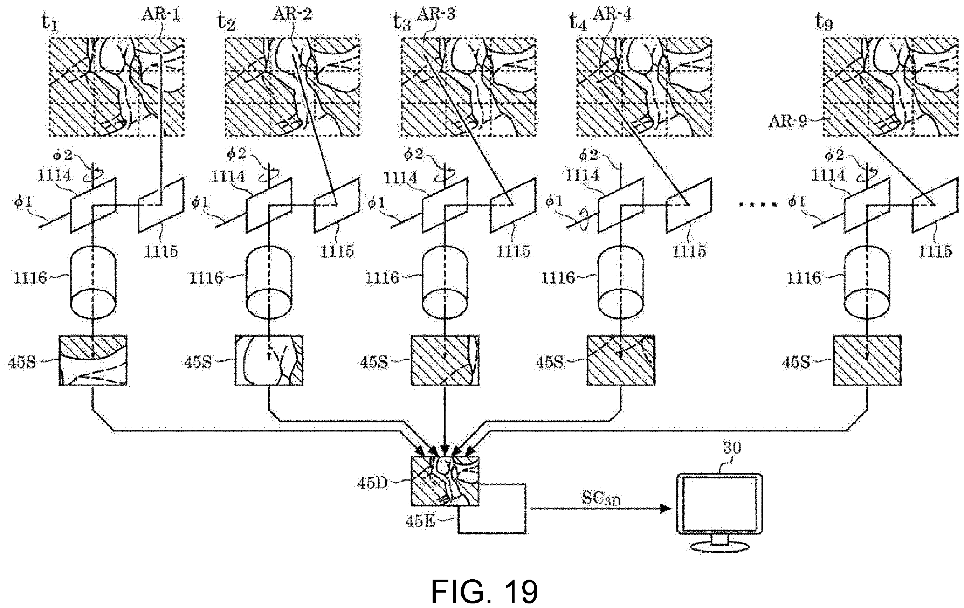

More specifically, as shown in FIG. 19, the control unit 46 divides the photographing range of the flexible endoscope 10' in the body cavity into M (M is a natural number of 2 or more, and M = 9 in the example of FIG. 19), and performs the following processing in accordance with the time t₁, time t₂, ... time t_{M(=9)} within the time T.

At the time t₁, the control unit 46 controls the tilt of the movable mirror 1114 so that the light of the first area AR-1 of the M areas AR-k (k = 1 to 9) obtained by dividing the entire photographing range into M is guided from the opening 1801 at the tip of the insertion part 110' to the multi-core fiber 1116 via the fixed mirror 1115 and the movable mirror 1114. The light of the area AR-1 reaches the solid-state imaging element 1311 through the multi-core fiber 1116. After photoelectric conversion in the solid-state imaging element 1311, the light of the area AR-1 is stored in the reception buffer 45S of the storage unit 45 as image data. When the image data of the area AR-1 is stored in the reception buffer 45S, the control unit 46 stores the image data in the storage area corresponding to the area AR-1 in the drawing frame buffer 45D.

At the time t₂, the control unit 46 controls the tilt of the movable mirror 1114 so that the light of the second area AR-2 of the M areas AR-k (k = 1 to 9) is guided from the opening 1801 at the tip of the insertion part 110' to the multi-core fiber 1116 through the fixed mirror 1115 and the movable mirror 1114. The light of the area AR-2 reaches the solid-state imaging element 1311 through the multi-core fiber 1116. After photoelectric conversion in the solid-state imaging element 1311, the light of the area AR-2 is stored in the reception buffer 45S of the storage unit 45 as image data. When the image data of the area AR-2 is stored in the reception buffer 45S, the control unit 46 stores the image data in the storage area corresponding to the area AR-2 in the drawing frame buffer 45D.

The control unit 46 performs the same processing at the time t₃, time t₄, time t₅, time t₆, time t₇, time t₈, and time t₉, and stores the image data generated for each of the area AR-3, area AR-4, area AR-5, area AR-6, area AR-7, area AR-8, and area AR-9 in a separate storage area of the drawing frame buffer 45D. According to the time of the next frame switching, the drawing frame buffer 45D is replaced with the display frame buffer 45E, and the image data of the areas AR-1, AR-2, AR-3, AR-4, AR-5, AR-6, AR-7, AR-8, and AR-9 in the display frame buffer 45E is output to the display device 30 as a moving image signal SC_{3D} of one frame.

The above are the details of the present embodiment. Here, the 8K solid-state imaging element can be housed in the housing 131 of the flexible endoscope 10' of the insertion part 110', but if the number of cores of the multi-core fiber 1116 is increased to be the same as the number of pixels of the solid-state imaging element, the flexibility may be impaired. The upper limit of the number of cores that can maintain the flexibility is about 10,000.

Regarding this, in the present embodiment, the movable mirror 1114 and the fixed mirror 1115 are provided in the flexible lens barrel 111' of the insertion part 110', and the movable mirror 1114 is tiltable around two axes, that is, the first axis ϕ1 having a tilt with respect to the optical axis direction of the light passing through each core CR of the multi-core fiber 1116, and the second axis ϕ2 orthogonal to the first axis ϕ1. Further, the control device 40 generates divided area images of different portions of the subject by periodically switching the tilt angles of the movable mirror 1114 at intervals of the time T shorter than the frame switching time interval of the frame rate of a moving image, and generates a moving image for one frame by combining the generated divided area images. Therefore, according to the present embodiment, an 8K photographed image can be obtained while the multi-core fiber 1116 is kept as thin as the conventional fiberscope (less than 2K). Thus, according to the present embodiment, the flexible endoscope 10' having an 8K resolution can be realized using a solid-state imaging element of less than 2K.

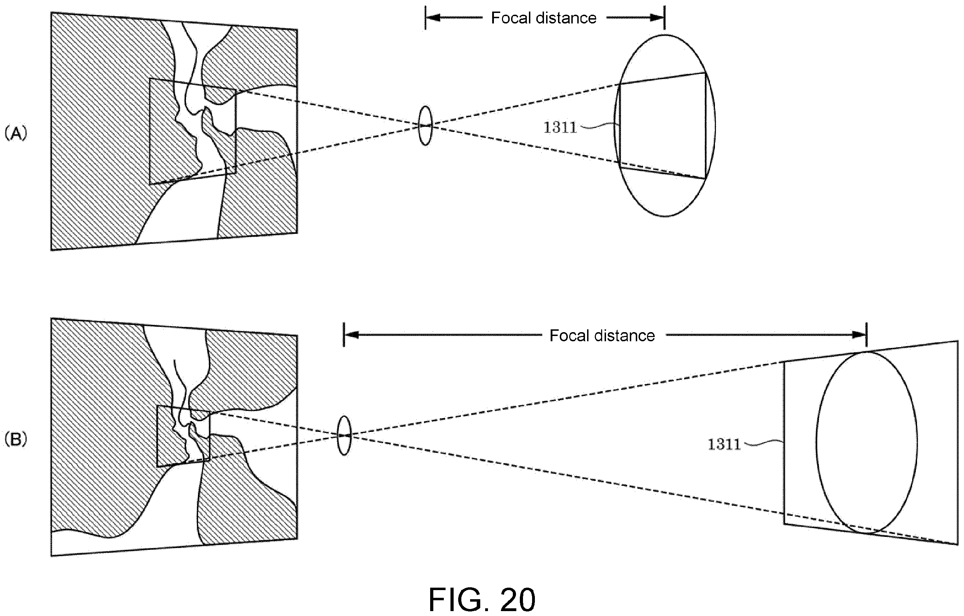

The first and second embodiments of the invention have been described above. Nevertheless, the following modifications may be added to the present embodiments.
(1) In the first and second embodiments, as shown in (A) of FIG. 20, the focal distance (distance between the solid-state imaging element 1311 and the optical system) may be set short so that the image circle of the optical system (objective lens 1111, eyepiece 1201, relay lens 1113, etc.) in the insertion part 110 of the endoscope circumscribes the light receiving area of the solid-state imaging element 1311, and as shown in (B) of FIG. 20, the focal distance (distance between the solid-state imaging element 1311 and the optical system) may be set long so that the image circle of the optical system (objective lens 1111, eyepiece 1201, relay lens 1113, etc.) in the insertion part 110 of the endoscope inscribes the light receiving area of the solid-state imaging element 1311.
(2) In the first and second embodiments, the position detection sensor 56 and the orientation detection sensor 57 are embedded in the polarized glasses 50, and the position detection sensor 36 and the orientation detection sensor 375 are also embedded in the display device 30. However, the display device 30 may not include the position detection sensor 36 and the orientation detection sensor 37. In that case, the control unit 46 may use fixed values of the X coordinate value, Y coordinate value, Z coordinate value, direction, and elevation angle of the position of the display device 30, and the detection signals of the detection signals of the position detection sensor 56 and the orientation detection sensor 57 of the polarized glasses 50 to generate the transformation matrix.
(3) In the first embodiment, 4320 rows and 7680 columns of the pixels PXij in the solid-state imaging element 1311 form blocks each having four pixels PXij in 2 rows and 2 columns, and red, green, blue, and near-infrared filters are affixed to the four pixels PXij of each block. However, red, green, blue, and near-infrared filters may not be affixed to each block of four as described above. For example, 4320 rows may divided into blocks every 4 rows, a red filter may be affixed to the pixels PXij in all columns in the first row of the blocks, a green filter may be affixed to the pixels PXij in all columns in the second row, a blue filter may be affixed to the pixels PXij in all columns in the third row, and near-infrared light may be affixed to the pixels PXij in all columns in the fourth row.
(4) In the first and second embodiments, the housing 131 has the buttons 139IN and 139OUT, and a trigger signal is generated when the buttons 139IN and 139OUT are pressed shortly once. However, the generation of the trigger signal may be triggered in other ways. For example, a microphone may be mounted on the housing 131, and the generation of the trigger signal may be triggered when the operator says the word "zoom". In addition, a zoom-in trigger signal that instructs zoom-in of the image displayed on the display device 30 may be generated when the button 139IN is pressed shortly once, and a release trigger signal that instructs to release the zoom-in may be generated and a zoom-out trigger signal may not be generated when the button 139IN is pressed long once.
(5) In the first and second embodiments, the solid-state imaging element 1311 is a CMOS image sensor. However, the solid-state imaging element 1311 may be constituted by a CCD (Charge Coupled Device) image sensor.
(6) In the second embodiment, one multi-core fiber 1116 is housed in the insertion part 110'. However, a plurality of multi-core fibers 1116 may be housed.
(7) In the second embodiment, the insertion part 110' has two mirrors, the fixed mirror 1115 and the movable mirror 1114. However, the number of mirrors may be one or three or more. Further, one or more of them may be movable mirrors. In short, the light needs to be guided from the subject to the multi-core fiber 1116 via one or a plurality of mirrors to make the scanning area variable.
(8) In the second embodiment, the first axis ϕ1 only needs to be tilted with respect to the optical axis of the light passing through each core of the multi-core fiber 1116, and does not necessarily intersect the optical axis of the light passing through each core of the multi-core fiber 1116. In this case, preferably the tilt of the movable mirror 1114 around the second axis ϕ2 is controlled so that the tilt in the first axis ϕ1 with respect to the optical axis direction of the light passing through each core CR of the multi-core fiber 1116 is 45 degrees ± a predetermined angle. In addition, preferably the tilt of the movable mirror 1114 about the first axis ϕ1 is controlled so that the tilt in the second axis ϕ2 with respect to the light passing through each core CR of the multi-core fiber 1116 is 90 degrees ± a predetermined angle.

### [Descriptions of Reference Numerals]

- 10: Rigid endoscope
- 10': Flexible endoscope
- 20: Illumination device
- 30: Display device
- 40: Control device
- 50: Polarized glasses
- 60: Air intake and exhaust device
- 70: Air cooling device
- 130: Housing
- 110: Insertion part
- 120: Eyepiece mount part
- 41: Wireless communication unit
- 42: Operation unit
- 43: Input/output interface
- 44: Image processing unit
- 45: Storage unit
- 46: Control unit

## Claims

1. An endoscope system, comprising:
an endoscope photographing a subject in a body cavity of a patient and outputting an image signal of a predetermined number of pixels;
a control device performing a predetermined 3D process on an output signal of the endoscope and outputting a 3D image signal obtained by the 3D process to a display device as a moving image signal of a predetermined frame rate;
polarized glasses worn by an operator performing a surgical operation on the patient;
sensors provided in the display device and the polarized glasses respectively; and
a trigger signal generating unit generating a trigger signal for instructing zoom of a display image of the display device,
wherein when the trigger signal generating unit generates the trigger signal, the control device identifies a fixation point of the operator in the display image of the display device based on a relationship between a detection signal of the sensor in the polarized glasses and a detection signal of the sensor in the display device at a time of generation of the trigger signal, and zooms in a periphery of the fixation point.

2. The endoscope system according to claim 1, wherein the sensors provided in the polarized glasses comprise a first sensor detecting a potential of a left nose pad of the polarized glasses, a second sensor detecting a potential of a right nose pad of the polarized glasses, a third sensor detecting a potential of a bridge of the polarized glasses, a fourth sensor detecting a position of the polarized glasses, and a fifth sensor detecting an orientation of lenses of the polarized glasses, and
the control device obtains a line-of-sight position corresponding to a line of sight of the operator on the lenses of the polarized glasses based on a potential waveform indicated by a detection signal of the first sensor, a potential waveform indicated by a detection signal of the second sensor, and a potential waveform indicated by a detection signal of the third sensor, and identifies the fixation point of the operator in the display image based on the line-of-sight position, a relationship between a position where the display device is placed and a position detected by the fourth sensor, and a relationship between an orientation of the display device and an orientation detected by the fifth sensor.

3. The endoscope system according to claim 2, wherein the endoscope is an 8K endoscope, comprising:
a housing;
a solid-state imaging element housed in the housing and comprising pixels, a number of which corresponds to 8K and which each comprise a photoelectric conversion element, arranged in a matrix; and
an insertion part extending with the housing as a base end, and the insertion part being inserted into the body cavity of the patient and guiding light from the subject in the body cavity to the solid-state imaging element,
wherein a pitch between adjacent pixels in the solid-state imaging element is larger than a longest wavelength of wavelengths of light in illumination that illuminates the subject.

4. The endoscope system according to claim 3, wherein the housing comprises a mount part having a large cross-sectional area orthogonal to an optical axis of light passing through the insertion part, and a grip part having a smaller cross-sectional area than the mount part, and
the solid-state imaging element is housed in the mount part.

5. The endoscope system according to claim 4, wherein the insertion part comprises a hollow rigid lens barrel, and
a plurality of lenses comprising an objective lens are provided in the rigid lens barrel.

6. The endoscope system according to claim 5, comprising:
an air supply pipe and an air exhaust pipe connected to the housing;
an air supply and exhaust device forcibly supplying air into the housing via the air supply pipe and forcibly exhausting air from the housing via the air exhaust pipe; and
an air cooling device cooling air flowing through the air supply pipe,
wherein the housing, the air supply pipe, and the air exhaust pipe are connected to form one closed space, and
in the housing,
a first heat sink provided on the solid-state imaging element; a FPGA for image processing, a second heat sink provided on the FPGA, and a cover member covering the second heat sink and connected to the air exhaust pipe are provided, and
in the housing, a first airflow for cooling the first heat sink and a second airflow for cooling the second heat sink are generated, wherein the first airflow is formed by blowing cooling air supplied from the air supply pipe to the first heat sink to diverge around the first heat sink, and the second airflow is formed to flow from around the second heat sink to the air exhaust pipe via the cover member.

7. The endoscope system according to claim 1, wherein the endoscope comprises:
a housing;
a solid-state imaging element housed in the housing and comprising pixels, which each comprise a photoelectric conversion element, arranged in a matrix; and
a hollow flexible lens barrel,
wherein in the flexible lens barrel, an objective lens, a multi-core fiber, and one or more mirrors for reflecting light from the subject one or more times and guiding the light to the objective lens are provided,
at least one mirror of the one or more mirrors is tiltable around two axes, a first axis having a tilt with respect to an optical axis direction of light passing through each core of the multi-core fiber, and a second axis orthogonal to the first axis, and
the control device generates divided area images of different portions of the subject by periodically switching a tilt angle of the mirror at a time interval shorter than a frame switching time interval of the frame rate, and generates a moving image for one frame by combining the generated divided area images.
